# EUROPEAN PATENT SPECIFICATION

(11) **EP 3 692 813 B1**
(45) Date of publication and mention of the grant of the patent: **13.12.2023**
(21) Application number: 18864057.7
(22) Date of filing: 25.09.2018
(51) Int. Cl.: A23L 31/00, A23K 10/16, A23L 33/135, A61K 35/74, A61P 3/02, C12N 1/20, A23K 10/18

(54) **COMPOSITION HAVING PHYSICAL STRENGTH IMPROVING EFFECT AND/OR ANTI-FATIGUE EFFECT**
ZUSAMMENSETZUNG MIT WIRKUNG ZUR VERBESSERUNG DER PHYSISCHE STÄRKE UND/ODER ANTIERMÜDUNG
COMPOSITION PRÉSENTANT UN EFFET D'AMÉLIORATION DE LA RÉSISTANCE PHYSIQUE ET/OU UN EFFET ANTI-FATIGUE

(30) Priority: 03.10.2017 JP 2017193132
(43) Date of publication of application: 12.08.2020
(73) Proprietor: Keio University, Tokyo 108-8345 (JP); Asahi Group Holdings, Ltd., Tokyo 130-8602 (JP)
(72) Inventor: FUKUDA, Shinji, Tsuruoka City Yamagata 997-0035 (JP); KANO, Chie, Sagamihara-shi Kanagawa 252-0206 (JP); MORITA, Hiroto, Sagamihara-shi Kanagawa 252-0206 (JP)
(74) Representative: Hoffmann Eitle
(86) International application number: PCT/JP2018/035295
(87) International publication number: WO 2019/069735

(56) References cited:
- EP-A1- 3 202 892
- WO-A1-2013/175038
- WO-A1-2016/049826
- WO-A1-2016/049879
- JP-A- 2009 545 314
- JP-A- 2015 023 875
- JP-A- 2015 503 340
- JP-A- 2016 516 419
- US-A1- 2015 216 913
- YI JU HSU et al.: "Effect of Intestinal Microbiota on Exercise Performance in Mice", the Journal of Strength and Conditioning Research, vol. 29, no. 2, February 2015 (2015-02), pages 552-558, XP055580222, DOI: doi:10.1519/JSC.0000000000000644
- Nahid SALARKIA et al.: "Effects of probiotic yogurt on performance, respiratory and digestive systems of young adult female endurance swimmers : a randomized controlled trial", Medical Journal of the Islamic Republic of Iran, vol. 27, no. 3, August 2013 (2013-08), pages 141-146, XP055589324,

## Description

### Technical Field

The present invention relates to use of *Bacteroides uniformis* strains JCM5828, CP3585 or CP3586 for improving one or more of physical strength and fatigue resistance.

### Background Art

For carrying out an activity such as labor or exercise, physical strength at least sustainable for such an activity is required in general. On the other hand, fatigue accumulates in the body through such an activity, which deteriorates body functions. It can be said that maintenance/improvement of physical strength and prevention/recovery of fatigue are indispensable for routinely carrying out an activity such as labor or exercise.

The physical strength required for carrying out the activity such as labor or exercise embraces physical abilities, such as muscular strength, endurance and flexibility, forming the fundamentals of action.

An amino acid composition said to improve endurance and to have an effect of preventing, reducing and recovering of fatigue has been developed/reported (Patent Literature 1).

Such an amino acid composition is, however, ingested before or during exercise, and exhibits the effect merely temporarily, and the effect cannot be expected depending on timing of ingestion in some cases. There is a demand for means capable of persistently exhibiting the effect without being affected by the timing of ingestion.

In addition, it has been reported that endurance improvement and an anti-fatigue effect are obtained by ingesting specific microorganisms or fermented composition.

Patent Literature 2 describes that a lactate production inhibitory effect, a blood lactate accumulation inhibitory effect, endurance improvement and an anti-fatigue effect can be obtained by ingesting a fermented composition (fermented liquid) obtained by inoculating natto bacteria in a medium containing rice brans and soybeans. Patent Literature 3 describes that an effect of increasing locomotor activity through fatigue accumulation preventing activity can be obtained by ingesting fermented soy milk obtained by lactic acid bacteria (*Leuconostoc pseudomesenteroides* strain ATCC 12291). These literatures describe the effects derived from the whole fermented compositions, and do not describe that the bacterial cells themselves exhibit the above-described effects.

Patent Literature 4 describes that endurance improvement and an anti-fatigue effect can be obtained by ingesting a bacterial cell powder of purple nonsulfur bacteria (*Rhodobacter azotoformans* strain BP0899).

Non Patent Literature 1 describes that endurance was improved in a mouse mono-associated with *Bacteroides fragilis* as compared with that in a germ-free mouse.

*Bacteroides uniformis* is a gram-negative anaerobic bacillus with a size of about 0.8 x 1.5 µm without forming a spore or having no motility. *Bacteroides uniformis* is an eubacteria usually present in the intestine of many mammals including a human.

### Citation List

### Patent Literature

Patent Literature 1: Japanese Patent Laid-Open No. 2008-255033
Patent Literature 2: Japanese Patent Laid-Open No. 2013-17445
Patent Literature 3: Japanese Patent Laid-Open No. 2008-179559
Patent Literature 4: WO2011/102035

### Non Patent Literature

Non Patent Literature 1: J. Strength Cond Res. 2015 Feb.; 29(2): 552-8

### Summary of Invention

### Technical Problem

It has not been cleared, however, what effect can be obtained by ingesting *Bacteroides uniformis.*

An object of the present invention is to provide means that can be ingested at arbitrary timing and can persistently exhibit one or more of a physical strength improving effect and an anti-fatigue effect.

### Solution to Problem

The present inventors made earnest studies to solve the above-described problems, and as a result, have found that the problems can be solved unexpectedly by ingestion of *Bacteroides uniformis,* strains JCM5828, CP3585 or CP3586, and thus, the present invention was accomplished.

Specifically, the present invention encompasses the following inventions:
[1] A composition containing *Bacteroides uniformis* or a treated product thereof, wherein the composition is used for improving one or more of physical strength and fatigue resistance, wherein Bacteroides uniformis is strain JCM5828, CP3585 or CP3586.
[2] The composition according to [1], wherein the composition is used for improving one or more of whole-body endurance and endurance in aerobic exercise.
[3] The composition according to any one of [1] to [3], wherein the composition is used as food/drink, a medicine, or feed or a feed additive.
[4] *Bacteroides uniformis* strain CP3585.
[5] *Bacteroides uniformis* strain CP3586.

### Advantageous Effects of Invention

The present invention can provide means that can be ingested at arbitrary timing and can persistently exhibit one or more of a physical strength improving effect and an anti-fatigue effect.

### Brief Description of Drawing

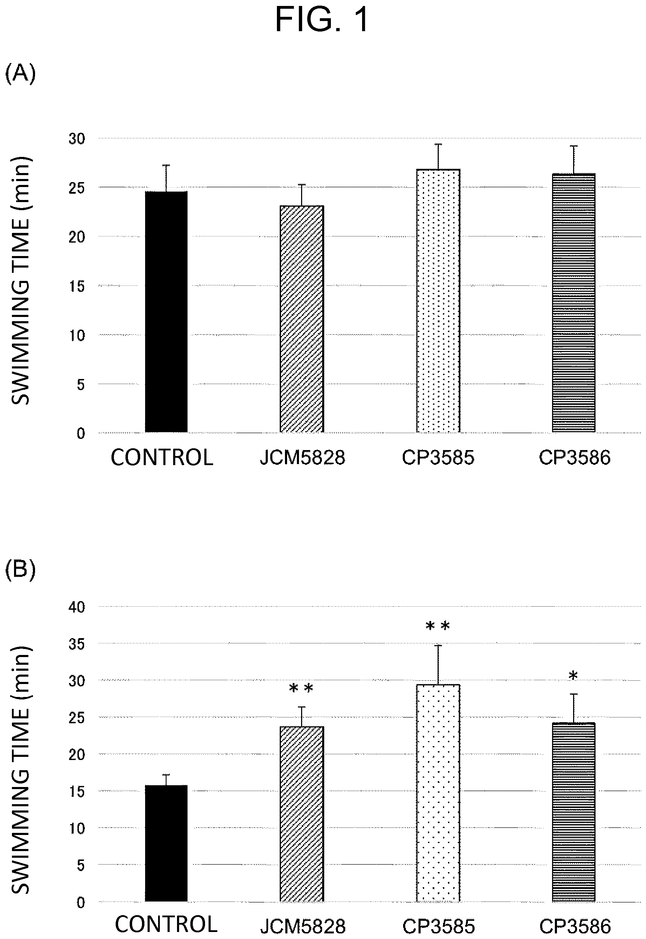

[Figure 1] Figure 1 illustrates graphs of results of forced swimming tests performed before starting administration of *Bacteroides uniformis* (Figure 1(A)) and 3 weeks after starting the administration (Figure 1(B)).

### Description of Embodiments

The present invention relates to a composition containing *Bacteroides uniformis* or a treated product thereof, wherein Bacteroides uniformis is strain JCM5828, CP3585 or CP3586, and having one or more of a physical strength improving effect and an anti-fatigue effect.

In the present invention, the term "physical strength" relates to physical abilities forming the fundamentals of action with which an activity such as labor or exercise can be carried out, and particularly means physical power with which an activity such as labor or exercise can be sustained and can be continuously carried out. More preferably, the term "physical strength" means one or more of whole-body endurance and endurance in aerobic exercise enabling aerobic exercise to be continuously done. The term "whole-body endurance" refers to ability generally designated as stamina with which physical activity can be continued for a long period of time.

In the present invention, the term "improvement of physical strength" means one or more of improvement and accelerated recovery of physical strength. In particular, it means one or more of improvement of the physical power with which an activity such as labor or exercise can be sustained and can be continuously carried out, and accelerated recovery of the physical power, and more preferably means improvement of one or more of the whole-body endurance and the endurance in aerobic exercise, and accelerated recovery of one or more of the whole-body endurance and the endurance in aerobic exercise.

In the present invention, the term "fatigue" means deterioration of body functions resulting from continuation of an activity such as labor or exercise.

In the present invention, the terms "anti-fatigue" and "improvement of fatigue resistance" mean at least one of reduction of fatigue, accelerated recovery of fatigue and prevention of fatigue. Preferably, these terms mean reduction of the deterioration of body functions resulting from the continuation of an activity such as labor or exercise, accelerated recovery of the deterioration of the body functions, or prevention of the deterioration of the body functions to attain a state where the body functions are difficult to deteriorate.

*"Bacteroides uniformis"* is a known bacterium, and can be characterized based on known mycological characteristics described in Bergey's Manual of Bacteriology, vol. 4 (1989) or the like.

In the present invention, *"Bacteroides uniformis"* can be one isolated from the intestine of a mammal (such as a human, a monkey, a chimpanzee, a bovine, a horse, a pig, sheep, a dog, a cat, a mouse or a rat), and preferably from a human.

*Bacteroides uniformis* can be isolated by a known method (Paola Gauffin Cano et al., PLoS One. July 2012, Volume 7, Issue 7, e41079). Specifically, feces, a fecal matter or stool of a mammal is diluted in an appropriate solvent, and the resultant is seeded in a plate medium to be cultured under anaerobic conditions, and bacteria are extracted from a colony having appeared in the medium. A medium described in detail later can be used, and from the viewpoint of efficiency of isolation, a selective isolation medium with which *Bacteroides* can be discriminated/selected is preferably used (Jap. J. vet. Sci., 36, 93-98 (1974)). After confirming that the obtained bacteria are gram-negative bacillus by gram staining and microscopic examination, the selected bacteria are analyzed based on the base sequence of 16S rRNA gene, and thus, *Bacteroides uniformis* can be identified/cloned. The base sequence of 16S rRNA gene of *Bacteroides uniformis* is known, and disclosed in, for example, known database such as GenBank, and registered as NR_040866, AB050110, AB510711, and L16486. These sequence information can be used in the analysis of the 16S rRNA gene. The analysis/identification of the 16S rRNA gene can be performed on the basis of a known method such as quantitative PCR, DGGE/TGGE, FISH, 16S rDNA cloning library, T-RFLP, FISH-FCM or base sequence determination (The Journal of Japanese Biochemical Society, vol. 80, No. 5, 421-425. 2008; J Nutr. 2004 Feb; 134 (2): 465-72; Appl. Environ. Microbiol. 64. 3336-3345. 1998; Appl. Environ. Microbiol., 65, 4799-4807, 1999; Appl. Environ. Microbiol., 62, 2273-2278, 1996; Appl. Environ. Microbiol., 64, 3854-3859, 1998). For example, when quantitative PCR is employed, on the basis of the known base sequence information of the 16S rRNA gene of *Bacteroides uniformis,* primers specific to the bacterium are prepared. A PCR reaction is performed using the primers with a DNA extracted from a selected bacterium used as a template, and it can be determined whether or not the bacterium is *Bacteroides uniformis* on the basis of presence of a PCR amplification product having an intended size. The specific primers can be designed and PCR conditions can be determined by ordinary methods (Baio Jikken Illustrated 3, Hontouni Fueru PCR: Saibou Kougaku Bessatsu, Me de Miru Jikken Note Series (Cell Technology, Separate Volume, Visual Laboratory Notebook Series); written by Hiroki Nakayama, Shujunsha Co., Ltd.).

Strain JCM5828, strain CP3585 and strain CP3586 are used in the present invention. Strain JCM5828 is a type strain registered/conserved in Microbe Division/Japan Collection of Microorganisms, RIKEN Bio Resource Research Center (305-0074 3-1-1 Takanodai, Tsukuba City, Ibaraki). Strain CP3585 and strain CP3586 are internationally deposited at Patent Microorganisms Depositary of National Institute of Technology and Evaluation (292-0818 Room 122, 2-5-8 Kazusakamatari, Kisarazu City, Chiba) with accession numbers NITE BP-02536 and NITE BP-02537 on August 25, 2017.

*Bacteroides uniformis* usable in the present invention can be cultured in a usual culture medium under culture conditions with which the bacterium can be cultured, and then collected.

The culture medium may be any medium in which *Bacteroides uniformis* can be cultured and is not especially limited, but for example, it can contain, as a carbon source, glucose, fructose, galactose, maltose, lactose, cellobiose, sucrose, rhamnose, amygdalin, esculin, salicin, melibiose, trehalose, L-arabinose, ribose, D-xylose, inulin, raffinose, starch, molasses or the like, as a nitrogen source, an inorganic ammonium salt such as ammonium sulfate or ammonium nitrate, or an organic nitrogen-containing substance such as urea, amino acid, a meat extract, a yeast extract, polypeptone or a proteolysis product, and as an inorganic salt, magnesium sulfate, potassium dihydrogen phosphate, potassium tartrate, zinc sulfate, magnesium sulfate, copper sulfate, calcium chloride, iron chloride, manganese chloride or the like. A known culture medium suitable for the culture of *Bacteroides uniformis* (such as a NBGT medium, a BL medium, or a GAM medium) can be used. The culture medium is preferably a liquid medium, and a solid medium or a semisolid medium can be used by addition of agar or gelatin if necessary.

The culture is performed at a temperature of 20°C to 50°C, preferably 25°C to 45°C, and more preferably 35°C to 37°C under anaerobic conditions. The "anaerobic conditions" may be a low oxygen environment in which *Bacteroides uniformis* can grow, and the anaerobic conditions can be attained by using, for example, an anaerobic chamber, an anaerobic box, a sealed vessel or a culture vessel holding an oxygen scavenger therein or the like.

The culture can be performed by any method such as static culture, shaking culture or tank culture, and a culture time is not especially limited, but can be, for example, 3 hours to 7 days.

After the culture, a resultant culture product may be directly used, or *Bacteroides uniformis* may be purified or crudely purified from the culture product before use. The purification or crude purification of bacterial cells from the culture product can be performed by any means, and for example, can be performed by centrifugation, filtration or the like.

*Bacteroides uniformis* used in the present invention may be live bacterial cells or dead bacterial cells, and may be wet bacterial cells or dried bacterial cells.

In the present invention, a treated product of *Bacteroides uniformis* can also be used. Examples of the "treated product" herein include bacterial cell debris and bacterial cell extracts of *Bacteroides uniformis.*

A bacterial cell debris can be obtained by subjecting bacterial cells to a known treatment such as a physical treatment, a chemical treatment, an enzyme treatment or autolysis, and the form of the bacterial cell debris is not limited as long as it has one or more of the physical strength improving effect and the anti-fatigue effect. The bacterial cell debris can be obtained by subjecting the bacterial cells to means, for example, using a homogenizer, a ball mill, a bead mill, a Dyno-mill, a planetary mill, a jet mill, a French press, a cell disruptor, or filtration. The physical disruption treatment may be performed by a wet method (treatment performed in a state of a bacterial cell suspension) or by a dry method (treatment performed in a state of a bacterial cell powder).

The bacterial cell extracts can be obtained by performing an extraction operation on the bacterial cells or the bacterial cell debris by using an extraction solvent, and collecting a fraction having one or more of the physical strength improving effect and the anti-fatigue effect. As the extraction solvent, for example, water, alcohol (such as ethanol or methanol), acetone, acetonitrile, dioxane, DMSO, DMF, diethyl ether, hexane, heptane, dichloromethane, chloroform, or a mixed solvent of any of these can be used. If necessary, the resultant bacterial extract may be concentrated by using a vaporizer such as an evaporator, or the fraction having one or more of the physical strength improving effect and the anti-fatigue effect can be further purified or crudely purified from the bacterial cell extracts. For further purifying or crudely purifying the fraction, a chromatography column, a filter, ultrafiltration, salting-out, solvent precipitation, solvent extraction, distillation, immunoprecipitation, polyacrylamide gel electrophoresis, isoelectric focusing, dialysis, recrystallization and the like can be appropriately selected and combined to be employed.

*Bacteroides uniformis* or a treated product thereof used in the present invention can be used in any form such as a dried product, a frozen product, a water dispersion or an emulsion. The dried product can be obtained, for example, by any drying means such as spray drying, drum drying, vacuum drying or freeze drying, and can be in a powdery, granular or similar form.

The composition of the present invention can contain, in addition to *Bacteroides uniformis* or a treated product thereof, an additive, such as an excipient, a lubricant, a binder or a disintegrating agent, usually used in production of a drug or food/drink, and can be produced in a dosage form or a form suitable to an intended administration route or ingestion method.

Examples of the excipient include lactose, white sugar, D-mannitol, D-sorbitol, starch, pregelatinized starch, dextrin, glucose, corn starch, crystalline cellulose, low substituted hydroxypropyl cellulose, sodium carboxymethyl cellulose, gum arabic, pullulan, light anhydrous silicic acid, synthetic aluminum silicate, and magnesium aluminometasilicate.

Examples of the lubricant include sugar esters such as sucrose fatty acid ester and glycerin fatty acid ester, hydrogenated oils such as calcium stearate, magnesium stearate, stearic acid, stearyl alcohol and powdered vegetable oil, waxes such as white beeswax, talc, silicic acid and silicon.

Examples of the binder include pregelatinized starch, sucrose, gelatin, gum arabic, methyl cellulose, carboxymethyl cellulose, sodium carboxymethyl cellulose, crystalline cellulose, white sugar, D-mannitol, trehalose, dextrin, pullulan, hydroxypropyl cellulose, hydroxypropyl methyl cellulose and polyvinyl pyrrolidone.

Examples of the disintegrating agent include lactose, white sugar, starch, carboxymethyl cellulose, calcium carboxymethyl cellulose, croscarmellose sodium, carboxymethyl starch sodium, light anhydrous silicic acid and low substituted hydroxypropyl cellulose.

Further, examples of additives usually used in production of a drug or food/drink and usable in the composition of the present invention include various fats and oils (for example, vegetable oils such as soybean oil, corn oil, safflower oil and olive oil, and animal fats such as beef tallow and sardine oil), crude drugs (for example, royal jelly and carrot), amino acid (for example, glutamine, cysteine, leucine and arginine), polyhydric alcohol (for example, ethylene glycol, polyethylene glycol, propylene glycol, glycerin, sugar alcohol such as sorbitol, erythritol, xylitol, maltitol and mannitol), natural polymers (for example, gum arabic, agar, water-soluble corn fiber, gelatin, xanthan gum, casein, gluten or a gluten hydrolysate, lecithin, starch and dextrin), vitamins (for example, vitamin C and vitamin B group), minerals (for example, calcium, magnesium, zinc and iron), dietary fiber (for example, mannan, pectin and hemicellulose), surfactants (for example, glycerin fatty acid ester and sorbitan fatty acid ester), purified water, a diluent, a stabilizer, a tonicity agent, a pH adjuster, a buffer, a wetting agent, a dissolution assisting agent, a suspending agent, a colorant, a flavoring agent, an odor improving agent, a perfume, an antioxidant, a sweetener, a taste component, and an acidulant.

In addition, the composition of the present invention can contain an additional component known to have one or more of the physical strength improving effect and the anti-fatigue effect. Examples of such an additional component include, but are not limited to, taurine, glutathione, carnitine, creatine, coenzyme Q10, glucuronic acid, glucuronolactone, guarana extract, theanine, γ-aminobutyric acid (GABA), capsaicin, capsiate, allysine, vitamins (such as vitamins B₁, B₂, B₆, B₁₂, C and E), various organic acids (such as citric acid), flavonoids, polyphenols, catechins and caffeine.

The dosage form or the form of the composition of the present invention is not especially limited. As a medicine, the composition can be in a dosage form of, for example, a tablet, a capsule, a granule, a powder, a dust formulation, a syrup, a dry syrup, a liquid, a suspension, an inhalant or a suppository, and is preferably in a dosage form of an oral agent. A liquid formulation such as a liquid or a suspension may be provided in a state where it can be freeze dried for storage, so as to be dissolved, in use, in water or a buffer containing a saline or the like to be prepared into an appropriate concentration for use. Alternatively, the composition in a solid dosage form such as a tablet may be coated if necessary (to obtain, for example, a sugar-coated tablet, a gelatin-encapsulated tablet or an enteric-coated tablet), or may be prepared, by known techniques, into a formulation controlled in release, such as a sustained release formulation, a delayed release formulation or an immediate release formulation.

Examples of the form of the food/drink include (but are not limited to) health food/drink (such as a supplement, a nutritional supplement, a dietary supplement and a balanced nutritional supplement) in the form of tablet confectionery, a tablet, a chewable tablet, a dust formulation, a powder, a capsule, a granule or a health drink, a refreshing drink, a tea drink, a jelly drink, a sports drink, a coffee drink, a carbonated drink, a vegetable drink, a fruit juice drink, a fermented vegetable drink, a fermented fruit juice drink, a fermented milk drink (such as yogurt), a lactic acid bacteria drink, a milk drink, a powdered drink, a cocoa drink, a sweet (such as a biscuit or cookie, a chocolate, a candy, a chewing gum or a tablet), and a jelly.

The food/drink can be food with health claims (food for specified health uses (including qualified food for specified health uses)), food with nutrient function claims, food with functional claims, health food or beauty health food, containing a component having one or more of the physical strength improving effect and the anti-fatigue effect.

In addition, the composition of the present invention can be in the form of not only food/drink for a human but also feed or a feed additive for a domestic animal, a racehorse, a pet or the like.

The amount of *Bacteroides uniformis* or a treated product thereof contained in the composition of the present invention can vary depending on the dosage form or form of the composition, and can be an amount, with respect to that of the composition, selected from a range usually of 0.0001 to 99% by mass, preferably of 0.001 to 80% by mass, and more preferably of 0.001 to 75% by mass. The amount of *Bacteroides uniformis* or a treated product thereof contained in the composition of the present invention is, for example, in terms of the number of bacterial cells, an amount, per 1 kg of the composition, selected from 1 x 10⁵ to 1 x 10¹⁷, preferably 1 x 10⁶ to 1 x 10¹⁶, and more preferably 1 x 10⁷ to 1 x 10¹⁵.

Examples of a subject (examinee) to administer or ingest the composition of the present invention include a mammal (such as a human, a primate (such as a monkey or a chimpanzee), a domestic animal (such as a bovine, a horse, a pig or sheep), a pet (such as a dog or a cat), an experimental animal (such as a mouse or a rat)), a bird and a reptile requiring one or more of the physical strength improvement and the anti-fatigue effect, and is preferably a mammal, and particularly preferably a human.

A dosage or an intake of the composition of the present invention can be changed in accordance with factors such as one or more of the age and the weight of a subject, the administration route, the number of times of administration/ingestion, and an extent of physical strength and fatigue, and an arbitrary dosage or intake can be employed. For example, for oral administration or ingestion, *Bacteroides uniformis* or a treated product thereof can be given or ingested in an amount, in terms of the number of bacterial cells, selected from 1 x 10⁵/kg to 1 x 10¹⁵/kg per day, preferably 1 x 10⁶/kg to 1 x 10¹⁴/kg per day, and more preferably 1 x 10⁸/kg to 1 x 10¹³/kg per day once or dividedly in plural times (of, for example, 2 to 5 times, and preferably 2 to 3 times) a day.

The composition of the present invention can achieve the effects in a very small amount and in a short period of time, but can be given or ingested for a long period of time. For example, the composition of the present invention can be given or ingested at the aforementioned dosage for 1 month or more, 2 months or more, 6 months or more, 1 year or more, or a longer period.

The composition of the present invention has one or more of the physical strength improving effect and the anti-fatigue effect. Specifically, the composition of the present invention exhibits, in a subject of the administration or ingestion as compared with a case where it is not given or ingested, effects of one or more of improvement and accelerated recovery of physical strength, particularly, one or more of improvement of physical power with which an activity such as labor or exercise can be sustained and can be continuously carried out and accelerated recovery of the physical powder, and more preferably improvement of one or more of the whole-body endurance and the endurance in aerobic exercise, accelerated of recovery of one or more of whole-body endurance and endurance in aerobic exercise, as well as one or more of reduction of fatigue, accelerated recovery of fatigue, and prevention of fatigue, and preferably one or more of reduction of deterioration of body functions resulting from continuation of an activity such as labor or exercise, accelerated recovery of the deterioration of the body functions, and prevention of the deterioration of the body functions to attain a state where the body functions are difficult to deteriorate. On the basis of these effects, the composition of the present invention is beneficial/effective for nutritional support and the like to fortify nourishment, or to overcome a weak constitution, physical fatigue or the like.

When the composition of the present invention is given to or ingested by the subject, one or more of the physical strength and the fatigue resistance of the subject can be improved as compared with a case where it is not given or ingested.

### Examples

The present invention will now be more specifically described, and it is noted that the present invention is not limited to these examples.

### Example 1: Extraction of Bacteroides uniformis

Human feces were diluted and seeded in a selective medium (NBGT agar medium), and cultured for 48 hours in an anaerobic state.

Next, each of colonies thus obtained was picked up and suspended in PBS, a part of the resultant suspension was seeded in a BL agar medium, and cultured for 48 hours in an anaerobic state. In addition, the rest of the suspension was used to extract DNA, and the DNA was used as a template for performing quantitative PCR and 16S rRNA gene sequencing to identify a strain of *Bacteroides uniformis.*

Each of the identified strains was collected from the BL agar medium, and inoculated/cultured in a GAM soft agar medium.

### Example 2: Preparation of Bacteroides uniformis Suspension

Each of two strains of *Bacteroides uniformis* obtained in Example 1 was applied onto a BL agar medium, and cultured for 48 hours. Subsequently, a colony obtained from each strain was picked up to a GAM liquid medium, and further cultured for 16 hours. The culture was performed all under anaerobic conditions. The obtained strains were respectively defined as "strain CP3585" and "strain CP3586".

After completing the culture, a supernatant was removed by centrifugation, and the collected bacterial cells were frozen at -80°C to be stored until administration.

Before administration, a suspension of the bacterial cells of each strain was prepared by dilution with PBS to 1 x 10⁹ CFU/ml.

In addition, strain JCM5828 corresponding to the type strain of *Bacteroides uniformis* was used similarly to prepare a suspension.

### Example 3: Effect for One or More of Physical Strength and Fatigue

### (1) Animal

Male C57BL/6 mice (5 weeks old) were used.

Since there is a difference in athletic ability among individual mice, a forced swimming test was performed once before administration, and mice were grouped into a control group, a strain JCM5828 treatment group, a strain CP3585 treatment group, and a strain CP3586 treatment group so that an average of maximum swimming times could be substantially the same among the groups (n in each group = 8).

The respective mice were raised under a specific-pathogen free (SPF) environment.

### (2) Administration of Bacterial Cells

One of the suspensions of strains JCM5828, CP3585 and CP3586 was forcedly orally given in an amount of 2 x 10⁸ CFU/day everyday over an experiment period (4 weeks).

The same amount of PBS was given to a control group.

### (3) Evaluation Method

The effect, obtained by the administration of the bacterial cells, for one or more of the physical strength and fatigue was evaluated by the forced swimming test (Journal of Japanese Society of Nutrition and Food Science, Vol. 62, No. 4, 165-170 (2009)). Specifically, a running water pool for mice was created by filling an acrylic water vessel having a length of 90 cm and a width of 45 cm with hot water up to a depth of 38 cm, and causing a current in a surface portion of the water using a pump connected thereto (a running water vessel for measuring physical exercise devised by Matsumoto, Kyoto University). When a mouse was put in the swimming machine, the mouse swum against the current, and sank in a rear portion of the water tank when exhausted. A swimming time from the start of the swimming to the sinking was measured. The forced swimming test was performed once a week over the experiment period (4 weeks).

### (4) Results

Results of the forced swimming tests performed before starting the administration (the forced swimming test performed for grouping the mice) and 3 weeks after starting the administration are illustrated in Figure 1. Figure 1 illustrates graphs of the results of the forced swimming tests performed before starting the administration of *Bacteroides uniformis* (Figure 1(A)) and 3 weeks after starting the administration (Figure 1(B)). The results of the control group are illustrated as "Control", and those of the treatment groups are illustrated as "strain JCM5828", "strain CP3585" and "strain CP3586". In each group, n = 8. **: p < 0.05, *: p < 0.1 (vs. control, Student T test)

In the results obtained before starting the administration (Figure 1(A)), there was no significant difference in the swimming time between the control group and the strain JCM5828, CP3585 and CP3586 treatment groups. On the other hand, in the results obtained 3 weeks after starting the administration (Figure 1(B)), the swimming time was found to be elongated in the strain JCM5828, CP3585 and CP3586 treatment groups as compared with that of the control group.

In addition, it was found that the swimming time of the control group became shorter in the results obtained after 3 weeks (Figure 1(B)) as compared with the results obtained before starting the administration (Figure 1(A)). This is probably because of fatigue accumulation over the long experiment period. On the other hand, such shortening was not found in the strain JCM5828, CP3585 and CP3586 treatment groups. This is one or more of the physical strength improving effect and the anti-fatigue effect of the given bacterial cells.

Based on these results, it was revealed that the administration of *Bacteroides uniformis* can elongate the duration of swimming (exercise), and provide the effects of improving physical strength and accelerating improvement or recovery of physical strength. It was also revealed that the administration of *Bacteroides uniformis* can suppress deterioration of body functions (fatigue) resulting from a continuous activity, and provide the effect of reducing or preventing fatigue, improving fatigue, or accelerating recovery.

It is noted that there was no significant difference, over the experiment period, in weight fluctuation between the control group and the treatment group of each type of bacterial cells.

## Claims

1. A composition for improving one or more of physical strength and fatigue resistance, which comprises *Bacteroides uniformis* or a treated product thereof, wherein *Bacteroides uniformis* is strain JCM5828 deposited under accession number ATCC 8492, CP3585 deposited under accession number NITE BP-02536 or CP3586 deposited under accession number NITE BP-02537.

2. The composition according to claim 1, wherein the composition is for improving one or more of whole-body endurance and endurance in aerobic exercise.

3. The composition according to claims 1 or 2, wherein the composition is for use as food/drink, a medicine, or feed or a feed additive.

4. *Bacteroides uniformis* strain CP3585 deposited under accession number NITE BP-02536.

5. *Bacteroides uniformis* strain CP3586 deposited under accession number NITE BP-02537.

## Patentansprüche

1. Zusammensetzung zum Verbessern einer oder mehrerer von Körperkraft und Ermüdungsbeständigkeit, umfassend *Bacteroides uniformis* oder ein behandeltes Produkt davon, wobei *Bacteroides uniformis* Stamm JCM5828, hinterlegt unter Hinterlegungsnummer ATCC 8492, CP3585, hinterlegt unter Hinterlegungsnummer NITE BP-02536, oder CP3586, hinterlegt unter Hinterlegungsnummer NITE BP-02537, ist.

2. Zusammensetzung gemäß Anspruch 1, wobei die Zusammensetzung zum Verbessern einer oder mehrerer von Ganzkörperausdauer und Ausdauer bei Ausdauertraining ist.

3. Zusammensetzung gemäß den Ansprüchen 1 oder 2, wobei die Zusammensetzung zur Verwendung als Lebensmittel/Getränk, als Medizin oder als Futtermittel oder Futtermittelzusatzstoff ist.

4. *Bacteroides uniformis* Stamm CP3585, hinterlegt unter Hinterlegungsnummer NITE BP-02536.

5. *Bacteroides uniformis* Stamm CP3586, hinterlegt unter Hinterlegungsnummer NITE BP-02537.

## Revendications

1. Composition d'amélioration d'une ou plusieurs caractéristiques parmi la force physique et la résistance à la fatigue, qui comprend *Bacteroides uniformis* ou un produit traité de celle-ci, dans laquelle *Bacteroides uniformis* est une souche JCM5828 déposée sous le numéro d'enregistrement ATCC 8492, CP3585 déposée sous le numéro d'enregistrement NITE BP-02536 ou CP3586 déposée sous le numéro d'enregistrement NITE BP-02537.

2. Composition selon la revendication 1, dans laquelle la composition sert à améliorer une ou plusieurs caractéristiques parmi l'endurance globale et l'endurance lors d'un exercice aérobique.

3. Composition selon la revendication 1 ou la revendication 2, dans laquelle la composition est destinée à être utilisée comme nourriture/boisson, médicament ou aliment ou additif alimentaire.

4. Souche CP3585 de *Bacteroides uniformis* déposée sous le numéro d'enregistrement NITE BP-02536.

5. Souche CP3586 de *Bacteroides uniformis* déposée sous le numéro d'enregistrement NITE BP-02537.
